# EUROPÄISCHE PATENTSCHRIFT

(11) **EP 3 556 451 B1**
(45) Veröffentlichungstag und Bekanntmachung des Hinweises auf die Patenterteilung: **03.06.2020**
(21) Anmeldenummer: 18168534.8
(22) Anmeldetag: 20.04.2018
(51) Int. Cl.: B01J 19/16, B01J 4/00, B01D 53/14, C07C 29/151, B01J 19/26

(54) **VERFAHREN ZUM BETREIBEN EINER REAKTORANLAGE**
METHOD FOR OPERATING A REACTOR SYSTEM
PROCÉDÉ DE FONCTIONNEMENT D'UNE INSTALLATION DE RÉACTEUR

(43) Veröffentlichungstag der Anmeldung: 23.10.2019
(73) Patentinhaber: Siemens Aktiengesellschaft, 80333 München (DE)
(72) Erfinder: Baldauf, Manfred, 91056 Erlangen (DE); Glöser, Lukas, 91052 Erlangen (DE); Stark, Katharina, 91052 Erlangen (DE); Tremel, Alexander, 91096 Möhrendorf (DE)

(56) Entgegenhaltungen:
- EP-A2- 0 430 699
- WO-A1-95/27690
- WO-A1-2016/128188
- WO-A1-2017/162512
- DE-A1-102015 202 680
- DE-A1-102015 215 662

## Beschreibung

Die Erfindung betrifft ein Verfahren zum Betreiben einer Reaktoranlage nach Patentanspruch 1.

Fossile Energieträger verursachen Kohlendioxidemissionen, die die Einhaltung globaler Klimaschutzziele erschweren. Aus diesem Grund wird der Ausbau erneuerbarer Energien vorangetrieben. Allerdings ist die Erzeugung regenerativen Stroms deutlich regionalen sowie zeitlichen Schwankungen unterworfen.

Beispielsweise wird bei sonnigen oder windigen Tagen durch Photovoltaik bzw. Windkraftanlagen Strom kostengünstig produziert. Derzeit wird nach wirtschaftlichen Ansätzen gesucht, diesen Strom über den Elektrizitätssektor hinaus sinnvoll zu nutzen und beispielsweise hieraus chemische Wertprodukte zu erzeugen. Eine Möglichkeit ist dabei die elektrochemische Umwandlung von Wasser in Wasserstoff und Sauerstoff. Der erzeugte Wasserstoff kann dann mit dem klimaschädlichen Kohlendioxid als Startmolekül bzw. Edukt reagieren, wodurch gleichzeitig Kohlendioxidemission verringert werden würde. Das relativ einfach verfügbare Kohlendioxid kann damit als kostengünstige Kohlenstoffquelle beispielsweise bei der Herstellung von Methanol als ein mögliches Produkt einer einstufigen Synthese von Kohlenstoffdioxid und Wasserstoff nach der Reaktionsgleichung

C0₂ + 3H₂ -> CH₃0H + H₂0

reagieren. Nachteilig an der Synthese von Methanol aus Kohlendioxid und Wasserstoff sind die niedrigen Gleichgewichtsumsätze, die in etwa nur 20 % bei 50 bar und 250°C betragen. Daher wird in herkömmlichen Syntheseanlagen ein großer Teil der gasförmigen Edukte im Kreis geführt, was zu erheblichen Druckverlusten im Reaktor und in Rohrleitungen führt und wodurch ein erheblicher Energieeintrag in Form von Kompressionsleistung und Wärmeleistung erfolgen muss. Desweiteren ist ein Gasrecycling wenig geeignet für einen dynamischen Betrieb einer Reaktionsanlage und kann deshalb auch schlecht an unregelmäßig auftretende elektrische Energiemengen angepasst werden, wie sie durch die fluktuierende Erzeugung auf Basis von Wind und Photovoltaik vorherrschen.

Ein Ansatz zur Umsetzung von gleichgewichtslimitierten Reaktionen wird bereits in der WO 2017212016 A1 beschrieben.

Hierbei kommt ein Rührkesselreaktor zum Einsetz, in dem ein Absorptionsmittel in einem unteren Bereich des Reaktors vorliegt und Eduktgase durch eine Katalysatoranordnung geleitet werden, wobei Produkte von dem Katalysator weit getrennt vorliegenden Absorptionsmittel aufgenommen werden. Eine derartige Anlage ist durchaus zweckdienlich, sie ist aber durch die Rühranordnung technisch aufwendig und weist Einschränkungen in der dynamischen Abfuhr des Produktes und der dynamischen Zufuhr von elektrischer Energie auf.

Weitere Verfahren zum Betreiben einer Reaktoranlage zur Umsetzung gleichgewichtslimitierter Reaktionen sind aus der DE 10 2015 202680 A1, WO2016/128188, EP 0430699 A2 und DE 10 2015 215662 A1 bekannt.

Bei der Prozessführung von Reaktoren zur Umsetzung gleichgewichtslimitierter Reaktionen der beschriebenen Art ist es häufig prozesstechnisch nicht vermeidbar, dass Edukte bis zum bestimmten Sättigungsgrad ebenfalls von dem Absorptionsmittel absorbiert werden. Es handelt sich dabei zwar nur um einen geringen Prozentsatz des Gesamteintrages der Edukte, der unter 10 %, in der Regel unter 5 % liegt, dennoch gehen diese Edukte bei der Entladung des Absorptionsmittels und bei der Trennung von den Produkten verloren. Hierdurch wird die Wirtschaftlichkeit des Gesamtprozesses beeinträchtigt.

Die Aufgabe der Erfindung besteht darin, ein Verfahren zum Betreiben einer Reaktoranlage zur Umsetzung gleichgewichtslimitierter Reaktionen bereitzustellen, dass gegenüber dem Verfahren aus dem Stand der Technik eine gesteigerte Wirtschaftlichkeit aufweist.

Die Lösung der Aufgabe liegt in einem Verfahren zum Betreiben einer Reaktoranlage mit den Merkmalen des Patentanspruchs 1.

Das erfindungsgemäße Verfahren zum Betreiben einer Reaktoranlage zur Umsetzung gleichgewichtslimitierter Reaktionen umfasst dabei folgende Schritte:
Zunächst werden Edukte (oder ein Edukt) zu einem (oder mehreren) Produkte in einer Reaktionskammer umgesetzt. Dabei herrscht im System ein Druck p1 vor. Da die Reaktion gleichgewichtslimitiert ist, was bedeutet, dass das Gleichgewicht nicht vollständig auf der Seite der Produkte liegt, wird ein Absorptionsmittel zum Einsatz gebracht, dass die Produkte, die bereits entstanden sind, aufnimmt beziehungsweise mit diesen beladen wird und aus der Reaktionskammer ausgeleitet wird. Beim Beladen des Absorptionsmittels mit den Produkten werden in unerwünschter Weise ebenfalls Edukte vom Absorptionsmittel mit aufgenommen. In einem zweiten Schritt wird das Absorptionsmittel, das im Wesentlichen die Produkte enthält aber auch mit einem geringen Anteil von Edukten beladen ist, aus der Reaktionskammer abgeführt. In einem weiteren Verfahrensschritt wird der Druck des Absorptionsmittels ausgehend vom Druck p1 auf einen Druck p2 abgesenkt. Der Druck p2 ist also kleiner als der Druck p1. Bei einem Absenken des Druckes wird das Absorptionsmittel von den Produkten und von den Edukten, die dann beide in Gasform vorliegen, befreit. Das Absorptionsmittel bleibt weiterhin in flüssiger Form vorliegend. In einem Teilschritt werden die gasförmigen Produkte durch Kondensation von gasförmigen Edukten getrennt. Sowohl die Produkte als auch die Edukte können dabei jeweils nur aus einer chemischen Komponente oder aus mehreren chemischen Komponenten bestehen. Dies ist abhängig von der gewählten Prozessführung und von der Reaktion an sich.

In einem anderen Teilschritt des Verfahrens wird das flüssige Absorptionsmittel mit einem Druck p3 beaufschlagt, wobei der Druck p3 größer ist als der Druck p1, der in der Reaktionskammer vorherrscht. Das flüssige Absorptionsmittel, das mit dem Druck p3 beaufschlagt ist, wird in einen Flüssigkeitsstrahlgaskompressor geleitet, in dem ebenfalls die Edukte, die bereits von den Produkten getrennt sind und in Gasform vorliegen, eingesaugt werden und in dem flüssigen Absorptionsmittel gelöst werden. Anschließend wird das flüssige Absorptionsmittel, das wiederum mit Edukten beladen ist, in die Reaktionskammer eingeführt.

Dadurch, dass in die Reaktionskammer ein Absorptionsmittel eingeleitet wird, das bereits mit den Edukten beladen wird, herrscht ein gewisser Sättigungsgrad des Absorptionsmittels für die Aufnahme von Edukten vor. Dies bedeutet wiederum, dass bei idealer Prozessführung keine weiteren Edukte während der Reaktion in das Absorptionsmittel gelangen können. Dabei wäre es grundsätzlich auch möglich, die Edukte, die von den Produkten getrennt und in Gasform vorliegen, zu komprimieren und wieder unter den notwendigen Reaktionsdruck p1 in die Reaktionskammer einzuleiten. Es ist jedoch energetisch günstiger und prozesstechnisch weniger aufwändig, das Absorptionsmittel, das ohnehin auf diesen Druck p1 angehoben werden muss, auf die Ebene eines Druckes p3 zu führen, der etwas höher liegt, als der Druck p1 und mit dem Druck p3 die gasförmigen Edukte mit einzusaugen und somit in der Absorptionsflüssigkeit zu lösen. Dies rührt daher, dass für die Beaufschlagung eine Flüssigkeit mit Druck weniger Energie aufgebracht werden muss, als dies für die Druckbeaufschlagung von Gasen notwendig ist, da Gase hoch kompressibel sind, was bei Flüssigkeiten nicht der Fall ist.

Der Druck p4, den das flüssige Absorptionsmittel mit dem darin gelösten Edukten aufweist, ist kleiner als der Druck p3, bevorzugt aber größer oder gleich dem Druck p1, sodass ohne weiterer Druckbeaufschlagung oder ohne weitere technische Maßnahmen das Absorptionsmittel wieder in die Reaktionskammer eingeleitet werden kann.

Der Vorteil des beschriebenen Verfahrens besteht darin, mit einem relativ geringen Energieaufwand die Edukte wieder in das Absorptionsmittel einzubringen, dieses mit den Edukten zu beladen, sodass eine Sättigung vorliegt und keine weiteren Edukte während des darauffolgenden Prozesses mehr aufnehmen kann, und somit keine weiteren Edukte dem Prozess verloren gehen. Die Edukte, die bereits in dem Absorptionsmittel geladen sind, liegen dort bevorzugt in einer gesättigten Form vor und sie werden in einem energiegünstigen Prozessschritt nach dem Entladen dem Absorptionsmittel wieder bis zu einem Sättigungsgrad zugeführt.

In einer weiteren Ausgestaltungsform der Erfindung wird der Druck des Absorptionsmittels nach Austreten aus der Reaktorkammer vom Druck p1 auf den Druck p2 in der Art abgesenkt, dass zwischen den Drücken p1 und p2 eine weitere Druckstufe vorliegt, die als p11 bezeichnet wird. Dabei wird sowohl für die Druckstufe p11, als auch für das Absorptionsmittel mit dem Druck p2 die Verfahrensschritte C, D und E gemäß Patentanspruch 1 ebenfalls durchgeführt. Das heißt auch bei der Zwischendruckstufe p11 wird zunächst der Teil der Produkte, die sich durch die Druckreduktion aus dem Absorptionsmittel entladen, durch Kondensation von den gasförmigen Edukten getrennt. Die analoge Durchführung des Verfahrensschrittes E des Patentanspruches 1 bedeutet dabei, dass das Absorptionsmittel, einen Druck aufweist, der höher als p11 ist, aber nicht notwendigerweise größer als p1 sein muss.

Grundsätzlich ist es natürlich zweckmäßig, neben der Druckstufe p11 auch noch weitere, kaskadenförmige Druckstufen zwischen der Druckstufe p1 und p2 einzufügen. Das hat den Vorteil, dass ausgehend von p2 der Druck des Absorptionsmittels sukzessive auf den Druck p4 wieder angehoben werden kann, also in mehreren Prozessschritten, was dazu führt, dass die Druckdifferenz, die der jeweilige Flüssigkeitsstrahlgaskompressor überbrücken muss, geringer ist, als wenn eine nur einstufige Erhöhung zwischen p2 und p4 erfolgen würde. Dies wiederum bedeutet, dass der Flüssigkeitsstrahlgaskompressor konstruktiv weniger anspruchsvoll und somit auch weniger kostenintensiv ausgelegt werden kann.

Der Druck p1, der in der Reaktorkammer vorherrscht und mit dem das Absorptionsmittel die Reaktorkammer verlässt, liegt je nach umzusetzender Reaktion und gewünschter Ausbeute und möglichen Energieeintrag zwischen 20 bar und 250 bar. Für eine Methanolsynthese aus den Edukten Kohlendioxid und Wasserstoff sind beispielsweise Drücke zwischen 50 bar und 85 bar in der Reaktorkammer als Druck p1 zweckmäßig. Dieser Druck p1 wird bis zum Druck p2, gegebenenfalls kaskadenförmig abgesenkt, wobei der Druck p2 zwischen 1 bar und 50 bar liegt, grundsätzlich erfolgte eine beste Entladung des Absorptionsmittel von Produkten und auch unerwünschten Edukten bei Drücken, die nahe am Atmosphärendruck, also zwischen 1 bar und 3 bar liegen.

Ferner ist es zweckmäßig, wenn der Druckunterschied zwischen den Drücken p1 und p11 und auch zwischen weiteren Zwischenstufen bis zum Druck p2 in einem Bereich liegt, der zwischen jeweils 3 bar und 10 bar aufweist, dies wiederum führt dazu, dass die Belastung des Flüssigkeitsstrahlgaskompressors gering gehalten werden kann. Auch die Pumpen, die das Absorptionsmittel von einer Druckstufe zur nächsten komprimieren, können mit entsprechend geringerem technischem Aufwand ausgelegt werden.

Weitere Ausgestaltungsformen und weitere Merkmale der Erfindung werden anhand der folgenden Figuren näher erläutert. Dabei handelt es sich um rein schematische und exemplarische Ausgestaltungsformen, die keine Einschränkung des Schutzbereiches darstellen.

Dabei zeigen,
- Figur 1: eine Reaktoranlage mit einer einstufigen Druckentlastung,
- Figur 2: eine Reaktoranlage mit einer mehrstufigen Druckentlastung und
- Figur 3: Querschnitt durch einen Flüssigkeitsstrahlgaskompressor.

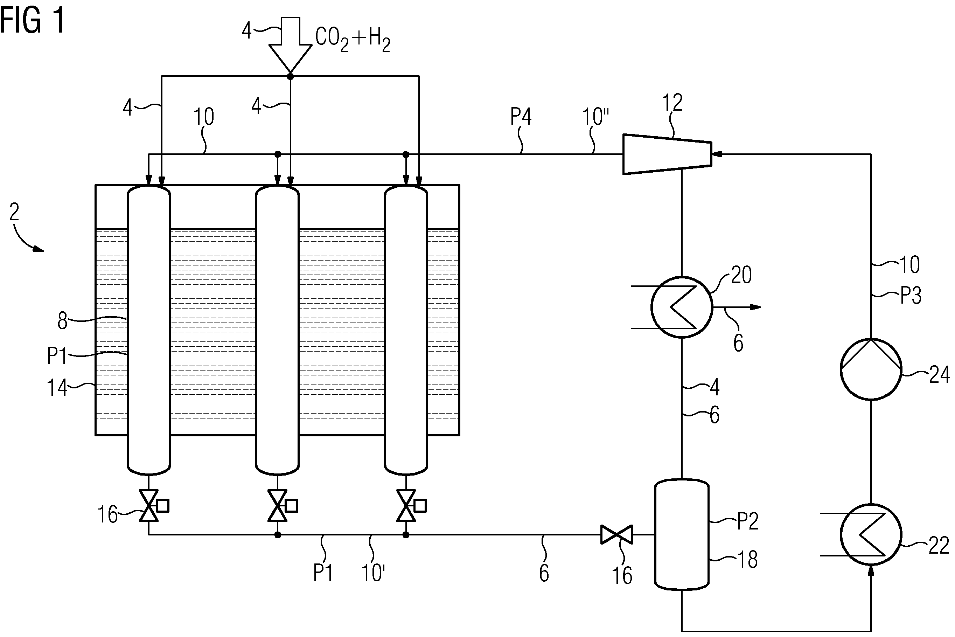

In Figur 1 ist zunächst schematisch eine Reaktoranlage 2 dargestellt, die beispielsweise auf der Basis eines Reaktorbündels 14 betrieben werden kann. Es sei im Folgenden kurz auf eine mögliche Funktionsweise einer derartigen Reaktoranlage, insbesondere zur Umsetzung von gleichgewichtslimitierten Reaktionen eingegangen. Gleichgewichtslimitierter Reaktionen, beispielsweise die Umsetzung von Kohlendioxid und Wasserstoff zu Methanol nach der Gleichung

CO₂ + 3H₂ -> CH₃OH + H₂O

weisen systembedingt aufgrund der Energiebilanz die Eigenschaft auf, dass das chemische Gleichgewicht mehr auf der linken Seite als auf der rechten Seite der Reaktionsgleichung liegt. In der beschriebenen Gleichung liegt das Gleichgewicht etwa zu 20 % auf der rechten Seite und zu 80 % auf der linken Seite. Dies führt dazu, dass für eine erfolgreiche Umsetzung der beschriebenen Gleichung möglichst das entstandene Produkt beziehungsweise die Produkte Methanol und Wasser aus dem Gleichgewicht zwischen den Edukten und den Produkten abgezogen werden sollten. Durch das Abziehen der Produkte wird das vorherrschende Gleichgewicht gestört, was zur Folge hat, dass das System bestrebt ist, wieder den Gleichgewichtszustand zu erreichen und hierzu Produkte gebildet werden.

Der Abtransport der Produkte geschieht zweckmäßigerweise durch ein Absorptionsmittel, beispielsweise durch eine ionische Flüssigkeit. Dieses Absorptionsmittel wird durch eine geeignete Prozessführung kontinuierlich im Durchfluss auch kontinuierlich mit den entstandenen Produkten beladen und aus der Reaktionszone herausgeführt. Das Absorptionsmittel 10 in der Reaktoranlage 2 weist dabei den vorherrschenden Prozessdruck p1 auf, wobei bei der beschriebenen Reaktion ein zweckmäßiger Prozessdruck p1 bei ca. 80 bar liegt. Neben den bereits beschriebenen Produkten Methanol und Wasser lösen sich in dem Absorptionsmittel 10 in unerwünschter Weise jedoch auch Spuren von Edukten, also von Kohlendioxid und Wasserstoff. Beide sind in Reinform Wertstoffe. Auch wenn die Lösung der Edukte im Absorptionsmittel je nach Prozessführung im kontinuierlichen Bereich lediglich zwischen 2 % und 5 % liegt, geht eben dieser Anteil der eingebrachten kostenintensiven Edukte verloren, wenn sie dem Prozess nicht wieder zurückgeführt werden. Daher wird im weiterhin beschriebenen Verfahren eine Möglichkeit vorgestellt, diese unerwünscht im Absorptionsmittel 10 gelösten Edukte 4 mit einem verhältnismäßig geringen Energieaufwand der Reaktoranlage wieder zuzuführen, und somit zu recyceln.

Das wie beschrieben beladene Absorptionsmittel 10 wird über ein Ventil 16 aus der Reaktorkammer 8 ausgeleitet. Sollte es sich, wie in Figur 1 dargestellt, um einen Reaktorbündel 14 handeln, so wird aus jeder einzelnen Reaktionskammer 8 des Reaktorbündels 14 das beladene Absorptionsmittel 10 über ein entsprechendes Ventil 16 ausgeleitet und gegebenenfalls über eine gemeinsame Leitung zu einer Druckkammer 18 geführt, wobei auf diesem Weg noch weitere Ventile 16 angeordnet sein können. In der Druckkammer 18 wird der Druck p1 des Absorptionsmittel, das im beladenen Zustand mit dem Bezugszeichen 10' versehen ist, entspannt, das heißt der Druck p1 wird auf einen Druck p2 abgesenkt. Je nach Beladungszustand und Prozessführung ist der Druck p2 deutlich geringer als der Druck p1. Liegt der Druck p1 im Bereich von etwa 80 bar, so liegt der Druck p2 näher am Atmosphärenbereich zwischen 1 bar und 5 bar, in der Regel nicht mehr als 10 bar. Durch die Druckentspannung des Absorptionsmittels 10' verliert es die Aufnahmefähigkeit für Produkte und Edukte, die sich dabei aus dem Absorptionsmittel 10' lösen und in gasförmiger Form entweichen. Das so entladene Absorptionsmittel 10 wird gegebenenfalls in eine Wärmetauschvorrichtung beziehungsweise Kühlvorrichtung 22 abgekühlt und durch eine Pumpe 24 wieder mit Druck beaufschlagt. Dieser Druck wird als p3 bezeichnet, wobei p3 größer ist als p2 und wie noch erläutert werden wird in dieser Ausgestaltungsform größer als p1 sein sollte. Für den Druck p3 ist gegebenenfalls einen Druck zu wählen, der im Bereich von 100 bar liegt.

In einem parallelen Prozessschritt wird das gasförmig entwichene Produkt 6, das in Gasform noch gemischt mit dem ebenfalls aus dem Absorptionsmittel 10 entladene Edukt 4 vorliegt, in einer Kondensationsvorrichtung 20 abgekühlt, wobei die Stoffe Wasser und Methanol, die in diesem Fall die Produkte 6 darstellen, in der Kühlvorrichtung kondensieren. Die Edukte 4, also das Kohlendioxid und der Wasserstoff kondensieren bei üblichen Kondensationsvorrichtungen nicht und bleiben in der Gasphase erhalten. Dies ist eine geeignete Trennmethode für die Trennung der unerwünschten vorhandenen Edukte und der werthaltigen Produkte 6.

Ohne der Bestrebung, den Produktionsprozess möglichst kostengünstig zu gestalten, könnten die Edukte 4 nun in die Umgebung abgegeben werden, es wäre jedoch auch möglich, diese durch einen Kompressor soweit zu verdichten, dass sie mit dem Prozessdruck p1 wieder der Reaktionskammer 8 zugeführt werden könnten. Da es sich bei den Edukten 4, die aus der Kondensationsvorrichtung 20 abgeschieden werden, jedoch um Gase handelt, müsste ein sehr hoher Energiebetrag eingebracht werden, um diese Gase wieder auf den Druck von ca. 80 bar, also den Druck p1 zu verdichten. Der Energieaufwand, der hierfür nötig wäre, würde je nach Rohstoffpreislage in etwa dem Wert entsprechen, den auch die Rohstoffe ohnehin bereits haben, sodass auch ein freies Entlassen der Edukte 4 aus der Kondensationsvorrichtung 20 in die Umgebung ähnlich wirtschaftlich beziehungsweise unwirtschaftlich wäre.

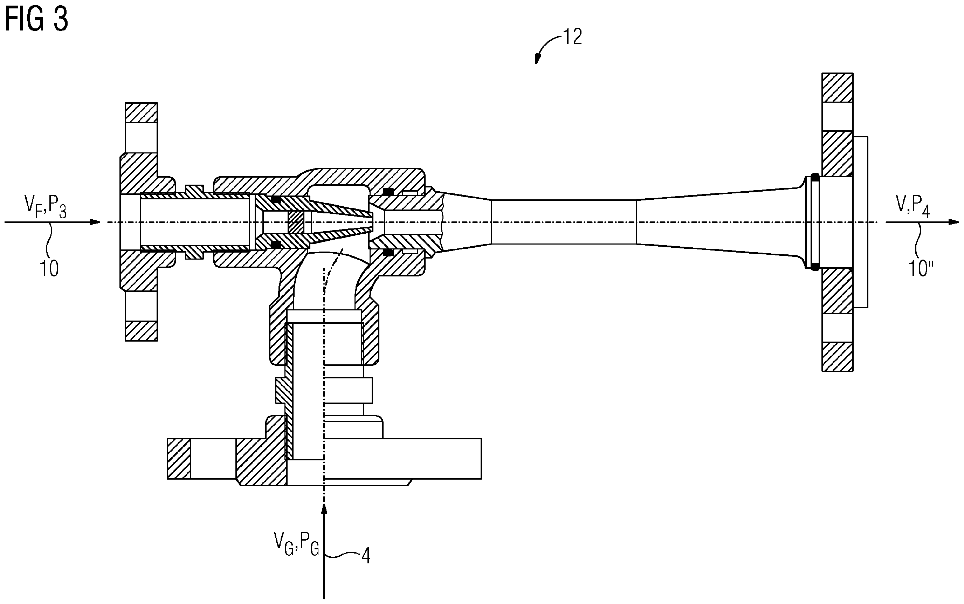

Nun ist dabei im vorliegenden Verfahren vorgesehen, das Absorptionsmittel 10, das nun den Druck p3 aufweist, einen sogenannten Flüssigkeitsstrahlgaskompressor 12 zuzuführen. Ein derartiger Flüssigkeitsstrahlgaskompressor funktioniert ähnlich wie eine Wasserstrahlpumpe in der Form, dass eine Flüssigkeit mit einem hohen Druck durch eine Düse geleitet wird und dabei über eine weitere Zuleitung ein Gas ansaugt. Schematisch ist ein Flüssigkeitsstrahlgaskompressor 12 in Figur 3 dargestellt. Dieser weist im Wesentlichen drei Öffnungen auf, eine der Öffnungen ist mit V_{F} bezeichnet, was für einen Volumenstrom V des Absorptionsmittels 10 steht. Das Absorptionsmittel 10 hat bei Eintritt in den Gaskompressor 12 einen Druck p3 und beim Austritt, der auf der rechten Seite dargestellt ist, einen Druck p4. Beim Austritt weist das Absorptionsmittel 10, das Bezugszeichen 10" auf, der Volumenstrom wird an dieser Stelle mit V bezeichnet. Der Druck p4 ist dabei geringer als der Druck p3. Dem Gaskompressor 12 wird durch eine dritte Öffnung Gas mit den Volumenstrom V_{G} (G für Gas) mit einem Druck pG eingeleitet wird. Dieses Gas enthält oder besteht im Wesentlichen aus den abgetrennten Edukte 4. Der Druck pG ist dabei nicht wesentlich größer als der Atmosphärendruck, was dazu führt, dass der Druck p3 auf den Druck p4 reduziert wird, wenn das Gas in Form der Edukte 4 dem Absorptionsmittel 10 im Gaskompressor 12 zugemischt wird.

Der Druck p4 ist dann wie bereits erwähnt entsprechend geringer als der Druck p3, wobei die Anlage entsprechend so ausgelegt ist, dass der Druck p4 in den Nähe des Drucks p1, sodass das mit den Edukten beladene Absorptionsmittel 10" möglichst ohne aufwendige Druckkorrektur wieder mit dem Druck p1 in die Reaktionskammer 8 eingespeist werden kann.

Der Vorteil des beschriebenen Verfahrens besteht darin, dass das entladene Edukt 4 beziehungsweise die Edukte 4 in Gasform vorliegen, und mit dem Druck pG, der in der Nähe des Atmosphärendruckes liegt, nicht wesentlich aufbereitet werden müssen und dem Absorptionsmittel 10 wieder beigemischt werden können. Es ist somit kein nennenswerter Energieeintrag für das Eduktgas 4 notwendig. Es ist lediglich ein Energieeintrag nötig, um das Absorptionsmittel 10 auf einen Druck p3 anzuheben, der etwas größer ist als der Prozessdruck p1. Der zusätzliche Energieaufwand besteht somit lediglich dadrin, die Druckdifferenz Δp zwischen p3 und p4 (Δp = p3 - p4) aufzubringen. Da es sich bei den Absorptionsmittel 10 um eine Flüssigkeit handelt, ist der Energieeintrag für eine Druckdifferenz zwischen beispielsweise 80 bar und 90 bar beziehungsweise 100 bar vergleichsweise gering. Der Energieeintrag in das Absorptionsmittel 10 zur Erzeugung von Δp ist zumindest deutlich geringer, als der Energieeintrag, der notwendig wäre, um das Edukt 4 in Gasform von Atmosphärendruck auf den Druck p1 anzuheben. Dieser eingesparte Energieaufwand ist letztendlich der Beitrag, der die Anlage gegenüber dem Stand der Technik wirtschaftlicher werden lässt.

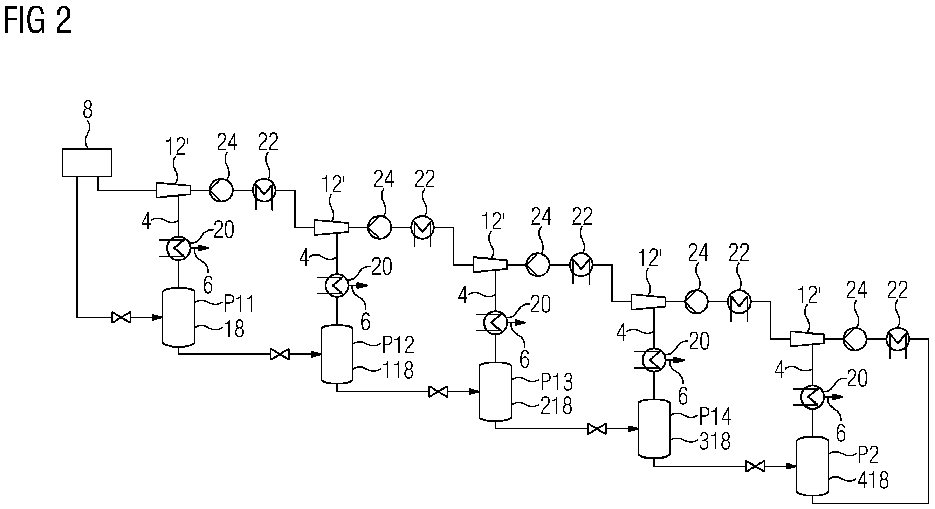

Wenn die Druckdifferenz zwischen p3 und p4 im Flüssigkeitsstrahl-Gaskompressor 12 reduziert werden würde, könnte der Gaskompressor 12 in einfacherer Weise ausgestaltet werden, was ebenfalls eine Kostenersparnis bedeutet. Das heißt ein kleineres Δp führt zu einer günstigeren Ausgestaltungsform des Gaskompressors 12. Dies führt zu der Ausgestaltungsform, die gemäß Figur 2 vorgestellt wird. Diese besteht darin, dass der Druck p1 des Absorptionsmittels 10 beziehungsweise 10' sukzessive, stufenweise über eine oder mehrere Kaskaden zum Druck p2 abgesenkt wird. Diese Zwischendrücke, die jeweils eine eigene Druckkammer 18 beziehungsweise 118, 218, 318 und 418 erfordern, liegen zwischen p1 und p2 und diese Zwischendrücke werden mit p11, p12, p13 und p14 bezeichnet. Der Druckabfall zwischen den jeweiligen Druckkammern 18 beziehungsweise 118 oder 218 usw. liegt in vorteilhafter Form bei etwa 5 bar, sodass in jeder Druckkammer 18, 118, 218 usw. ein Teil der Produkte 6 beziehungsweise auch der Edukte 4 aus dem Absorptionsmittel 10' entladen werden. Es erfolgt in keiner der genannten Druckkammern 18 eine vollständige Entladung des Absorptionsmittels 10', sondern lediglich eine Teilentladung. Erst in der letzten Druckkammer, nach dieser Diktion, 418 wird das Absorptionsmittel 10' auf den Enddruck und Entspannungsdruck p2 abgesenkt und dort vollständig entladen.

Die bereits beschriebenen Prozessschritte des Kondensierens der Produkte 6 in einer Kondensationsvorrichtung 20 und das Einspeisen der nichtkondensierten Edukte 4 in den jeweiligen Flüssigkeitsstrahl-Gaskompressor erfolgt in analogerweise, wie bereits in Figur 1 beschrieben. Grundsätzlich werden bei dieser kaskadenförmigen Anordnung entsprechend mehr Einzelkomponenten benötigt als in der Darstellung gemäß Figur 1, dafür kann der jeweilige Flüssigkeitsstrahl-Gaskompressor 12 und die jeweilige Pumpe 24 jedoch deutlich günstiger ausgestaltet werden, als der Gaskompressor 12 und die Pumpe 24 gemäß Figur 1. Welche Methode letztendlich die Wirtschaftlichere ist, hängt sehr stark von der Prozessführung und von den einzelnen Kosten für die einzelnen Prozesskomponenten ab.

### Bezugszeichenliste

- 2: Reaktoranlage
- 4: Edukte
- 6: Produkte
- 8: Reaktionskammer
- 10: Absorptionsmittel
- 10': beladenes Absorptionsmittel
- 10": Absorptionsmittel
- 12: Flüssigkeitsstrahl-Kompressor
- 14: Reaktorbündel
- 16: Ventile
- 18: Druckkammer 118, 218, 318, 418
- 20: Kondensationsmittel
- 22: Kühlvorrichtung
- 24: Pumpe
- V_{F}: Volumenstrom Absorptionsmitteleinlass
- V_{G}: Volumenstrom Gase
- V: Volumenstromgemisch
- p1: Druck
- p2: Druck
- p3: Druck
- p4: Druck
- P11: Druck

## Patentansprüche

1. Verfahren zum Betreiben einer Reaktoranlage (2) zur Umsetzung gleichgewichtslimitierter Reaktionen, umfassend folgende Schritte:
- Umsetzen von Edukten (4) zu einem Produkt (6) in einer Reaktorkammer (8) bei einem Druck p1, wobei ein Absorptionsmittel (10) mit dem Produkt (6) beladen wird und dabei ebenfalls Edukte (4) aufnimmt,
a) Ausleiten des beladenen Absorptionsmittel (10') aus der Reaktorkammer (8), und
b) Absenken des Drucks des Absorptionsmittels (10) auf einen Druck p2, wobei der Druck p2 kleiner als der Druck p1 ist, und wobei das Produkt (6) und Edukte (4) in Gasform von dem flüssigen Absorptionsmittel (10) entladen werden und
c) die gasförmigen Produkte (6) durch Kondensation von den gasförmigen Edukten (4) getrennt werden,
d) während das flüssige Absorptionsmittel (10) mit einen Druck p3 beaufschlagt wird, wobei der Druck p3 größer ist als der Druck p1 und
e) das flüssige Absorptionsmittel (10) mit dem Druck p3 in einen Flüssigkeitsstrahl-Gaskompressor (12) geleitet wird, in dem die von den Produkten (6) getrennten, gasförmigen Edukte (4) angesaugt werden und in dem flüssigen Absorptionsmittel (10) gelöst werden und
f) anschließend mit dem Druck p4, der kleiner als der Druck p3 ist, in die Reaktorkammer (8) eingeführt werden.

2. Verfahren nach Anspruch 1, **dadurch gekennzeichnet, dass** der Druck des Absorptionsmittels (10) von dem Druck p1 auf den Druck p2 über eine Druckstufe p11 abgesenkt wird und die Verfahrensschritte c), und e) analog auch für den Druck p11 durchgeführt werden.

3. Verfahren nach Anspruch 1 oder 2, **dadurch gekennzeichnet, dass** der Druck p1 zwischen 20 bar und 250 bar liegt.

4. Verfahren nach einem der vorhergehenden Ansprüche, **dadurch gekennzeichnet, dass** der Druck p2 zwischen 1 bar und 50 bar liegt.

5. Verfahren nach einem der Ansprüche 2 bis 4, **dadurch gekennzeichnet, dass** der Druck p11 zwischen 3 bar und 10 bar kleiner ist als der Druck p1.

## Claims

1. Method for operating a reactor system (2) for carrying out equilibrium-limited reactions, comprising the following steps:
- converting starting materials (4) into a product (6) in a reaction chamber (8) under a pressure p1, wherein an absorbent (10) is laden with the product (6) and thereby also absorbs starting materials (4),
a) discharging the laden absorbent (10') from the reaction chamber (8), and
b) lowering the pressure of the absorbent (10) to a pressure p2, wherein the pressure p2 is lower than the pressure p1,
and wherein the product (6) and starting materials (4) are discharged in a gaseous state from the liquid absorbent (10) and
c) the gaseous products (6) are separated from the gaseous starting materials (4) by condensation,
d) while a pressure p3 is applied to the liquid absorbent (10), the pressure p3 being higher than the pressure p1, and
e) the liquid absorbent (10) is conducted under the pressure p3 into a liquid-jet gas compressor (12), in which the gaseous starting materials (4) separated from the products (6) are aspirated and dissolved in the liquid absorbent (10) and
f) are then introduced into the reaction chamber (8) under the pressure p4, which is lower than the pressure p3.

2. Method according to Claim 1, **characterized in that** the pressure of the absorbent (10) is lowered from the pressure p1 to the pressure p2 over a pressure stage p11 and method steps c) and e) are also carried out analogously for the pressure p11.

3. Method according to Claim 1 or 2, **characterized in that** the pressure p1 lies between 20 bar and 250 bar.

4. Method according to one of the preceding claims, **characterized in that** the pressure p2 lies between 1 bar and 50 bar.

5. Method according to one of Claims 2 to 4, **characterized in that** the pressure p11 is between 3 bar and 10 bar lower than the pressure p1.

## Revendications

1. Procédé pour faire fonctionner une installation (2) de réacteur pour effectuer des réactions limitées à l'équilibre, comprenant les stades suivants :
- transformation d'éduits (4) en un produit (6) dans une chambre (8) de réacteur, sous une pression p1, dans lequel on charge du produit (6) à un agent (10) d'absorption et on absorbe ainsi également des éduits (4),
a) on sort l'agent (10') d'absorption chargé de la chambre (8) de réacteur, et
b) on abaisse la pression de l'agent (10) d'absorption à une pression p2, la pression p2 étant plus basse que la pression p1, et on décharge le produit (6) et les éduits (4) sous forme gazeuse de l'agent (10) d'absorption liquide et
c) on sépare des éduits (4) gazeux les produits (6) gazeux par condensation,
d) pendant que l'on soumet l'agent (10) d'absorption liquide à une pression p3, la pression p3 étant plus haute que la pression p1 et
e) on envoie l'agent (10) d'absorption liquide ayant la pression p3 dans un compresseur (12) de gaz à jet de liquide, dans lequel on aspire les éduits (4) gazeux séparés des produits (6) et on les dissout dans l'agent (10) d'absorption liquide et
f) ensuite, à la pression p4, qui est plus basse que la pression p3, on les introduit dans la chambre (8) de réacteur.

2. Procédé suivant la revendication 1, **caractérisé en ce que** l'on abaisse la pression de l'agent (10) d'absorption, de la pression p1 à la pression p2, par un échelon p11 de pression et on effectue les stades c) et e) du procédé de la même façon également pour la pression p11.

3. Procédé suivant la revendication 1 ou 2, **caractérisé en ce que** la pression p1 est comprise entre 20 bar et 250 bar.

4. Procédé suivant l'une des revendications précédentes, **caractérisé en ce que** la pression p2 est comprise entre 1 bar et 50 bar.

5. Procédé suivant l'une des revendications 2 à 4, **caractérisé en ce que** la pression p11, comprise entre 3 bar et 10 bar, est plus petite que la pression p1.
